# EUROPÄISCHE PATENTSCHRIFT

(11) **EP 2 088 973 B1**
(45) Veröffentlichungstag und Bekanntmachung des Hinweises auf die Patenterteilung: **30.04.2014**
(21) Anmeldenummer: 08758896.8
(22) Anmeldetag: 30.05.2008
(51) Int. Cl.: A61F 5/14

(54) **INNENSOHLE MIT VERSTEIFUNGSELEMENT**
INSOLE WITH REINFORCEMENT ELEMENT
SEMELLE INTÉRIEURE COMPORTANT UN ÉLÉMENT DE RIGIDIFICATION

(30) Priorität: 19.09.2007 DE 202007013120 U
(43) Veröffentlichungstag der Anmeldung: 19.08.2009
(73) Patentinhaber: Cetec AG, FL-9490 Vaduz (LI)
(72) Erfinder: AHLBÄUMER, Georg, FL-9490 Vaduz (LI)
(74) Vertreter: Grünecker, Kinkeldey, Stockmair & Schwanhäusser
(86) Internationale Anmeldenummer: PCT/EP2008/004325
(87) Internationale Veröffentlichungsnummer: WO 2009/039902

(56) Entgegenhaltungen:
- WO-A-2004/023916
- DE-U1- 20 314 288
- US-A- 5 404 659
- US-B1- 6 301 807

## Beschreibung

Die vorliegende Erfindung bezieht sich auf eine Innensohle, die im Wesentlichen dem Profil eines menschlichen Fußes angepasst ist und im Mittelfußbereich eine dem Fuß zugewandte kuppelartige, elastisch verformbare Wölbung aufweist, wobei sich auf der dem Fuß abgewandten Seite der In nensohleim Bereich der Wölbung ein Versteifungselement befindet, welches aus einem Material mit höherer Steifigkeit als das Material der Innensohle besteht, und eine konvexe Form aufweist.

Innensohlen, insbesondere Einlegesohlen, für Schuhe besitzen vielfältige Funktionen. Sie verbessern den Tragekomfort von Schuhen, z.B. durch Auspolsterung der der Fußunterseite zugewandten Stellen oder das Vorsehen von dämpfenden Elementen. Auch werden sie im medizinischen Bereich eingesetzt, um Fehler im Gang eines Menschen zu korrigieren oder um den Fuß zu entspannen oder zu stabilisieren.

Auch gibt es Innensohlen, die das Schuhinnere entlüften, um so unangenehmen Geruchsentwicklungen im Schuhinneren entgegenzuwirken. Besonders bei Sportlern und Menschen mit starker Schweißbildung führen die menschlichen Ausdünstungen im Schuhinneren zu einer möglichen starken Geruchsentwicklung, die mit einer entsprechenden Entlüftung des Schuhinneren weitgehend kompensiert werden kann.

Zahlreiche Patentschriften beschäftigen sich aufgrund der beschriebenen allgegenwärtigen Problematik mit der Ausbildung von speziellen Schuhinnensohlen, die den Tragekomfort von Schuhen erhöhen oder medizinischen Zwecken dienen.

Beispielsweise sind in den Patentschriften US-5,404,659 und US-6,301,807 Innensohlen beschrieben, die durch eine ausgefüllte bzw. mit Stützelementen versehene Wölbung der Innensohle beim Träger eines Schuhes mit einer solchen Innensohle einen propriozeptiven Effekt bewirken und so einen Fuß in seiner Struktur stärken, wodurch z.B. das Verletzungsrisiko verringert wird.

Zahlreiche Patentschriften und Anmeldungen beschäftigen sich mit der Ventilation des Schuhinneren. Beispielsweise zeigen die Anmeldungen JP-11032809A und JP-2000106908A die Innenbelüftung von Schuhen, wobei die Belüftung in beiden Fällen durch eine Pumpe, die in der Innensohle integriert ist, bewirkt wird. In der Anmeldeschrift JP-2000106908A weist die Belüftung noch zusätzlich eine an die Pumpe angeschlossene Leitung mit Belüftungsöffnungen auf, durch welche die Luft in der Pumpe bei der Kompression dieser in den Schuhinnenraum gepumpt wird. Des Weiteren befinden sich in der Pumpe Zypressenschnitzel, welche die Belüftungsluft aromatisieren sollen.

FR-A-2 395 719 offenbart eine Innensohle, die im Wesentlichen dem Profil eines menschlichen Fußes angepasst ist und im Bereich des Mittelfußes eines dem Fuß zuwandte kuppelartige, elastisch verformbare Wölbung mit Entlüftungsöffnung.

EP-0 903 984-B1 zeigt eine Ausführung einer Schuhinnenbelüftung, bestehend aus einer Schuhaußensohle, einer Innensohle und einer entsprechenden Mittelsohle. Dabei weist die Erfindung zwischen Mittelsohle und Schuhaußensohle im vorderen Fußbereich Luftkammern auf, deren Luft bei der Fortbewegung durch die Verformung dieser Bereiche durch Löcher in der Innensohle im vorderen Fußbereich in das Schuhinnere geleitet wird.

EP-1 536 710-A1 zeigt eine Einlegesohle aus elastischem Kunststoffmaterial mit elastisch verformbaren Wölbung mit eingestanzten Entlüftungsöffnungen und strahlenförmig ausgeführte Rillen auf der Unterseite, über die aus dem Schuhinneren ventilierte Luft gegen Außenluft ausgetauscht wird.

Da es sich bei einer Schuhinnensohle um einen Massenartikel handelt, ist es insbesondere von Bedeutung, den technischen sowie den finanziellen Herstellungsaufwand eines solchen Artikels so gering wie möglich zu halten. Gleichzeitig soll der Artikel eine gleichbleibende, hohe Qualität aufweisen.

Um diese Ziele zu erreichen, ist es die Aufgabe der vorliegenden Erfindung, die aus EP-1 536 710-A1 bekannte Innensohle so zu verbessern, dass ein Produkt mit langer Lebensdauer geschaffen wird, welches über die Lebensdauer eine gleichbleibende Tragequalität aufweist und welches in Verbindung mit einem geeigneten Schuh den Tragekomfort verbessert indem es unter anderem das Schuhinnere be- bzw. entlüftet.

Diese Aufgabe wird in erfinderischer Weise durch den Gegenstand des Anspruchs 1 gelöst. Vorteilhafte Ausgestaltungen der Erfindung sind Gegenstand mehrerer Unteransprüche.

Die vorliegende Erfindung baut auf der Erkenntnis auf, dass sich durch eine Ausbildung einer dem Fuß zugewandten, kuppelartigen, elastisch verformbaren Wölbung der Tragekomfort eines Schuhes mit erfindungsgemäßer Innensohle erheblich verbessern lässt. Durch die Ausbildung einer Wölbung auf der Oberseite der Innensohle liegt die Innensohle im gesamten Bewegungsablauf beim Gehen an der Fußunterseite an. Dadurch erhöht sich subjektiv der Tragekomfort für den Träger eines Schuhes mit erfindungsgemäßer Innensohle. Befindet sich die Wölbung der Innensohle im Mittelbereich des Fußes, so lassen sich die bei der Fortbewegung wirkenden Kräfte besonders vorteilhaft für die Verformung der Wölbung und somit für die Luftzirkulation (Ventilation) im Schuh einsetzen.

Um Sicherzustellen, dass der gewünschte Tragekomfort lange anhält ist in erfindungsgemäßer Weise vorgesehen, die Innensohle mit einem Einlegeteil zur Versteifung auszustatten, welches entweder lose eingelegt wird oder mit der Innensohle fest verbunden ist.

Die Form des Einlegeteils ist vorzugsweise der Wölbung der Innensohle angepasst. Die Grundfläche der Wölbung beschreibt eine Ellipse, im Sinne einer gleichmäßigeren Kräfteverteilung hat es sich jedoch als besonders vorteilhaft erwiesen, die Grundfläche des Versteifungselementes in Knochenform auszuführen.

Die Knochenform des Einlegeteils kann unsymmetrisch ausgeführt sein, so dass z.B. die der Fußspitze zugewandte Seite des Knochen verbreitert wird bzw. die dem Fußende zugewandte Seite verschmälert wird um so den anatomischen Merkmalen des Fußes und der darauf in der Form angepassten Innensohle Rechnung zu tragen.

Zur Unterstützung der federelastischen Eigenschaften des Einlegeteils hat es sich als vorteilhaft erwiesen, dass der eingesetzte Kunststoff im Vergleich zur dem bei der Innensohle verwendeten Kunststoff eine höhere Steifigkeit aufweist. Zusätzlich kann man durch nachträgliches Einbringen oder vorheriges Aussparen von Schlitzen die federelastischen Eigenschaften des knochenförmigen Einlegeteils verbessern.

Durch die Verformung der im Mittelfußbereich ausgeprägten unausgefüllten Wölbung der Innensohle, bewirkt durch Gehen, wird Luft durch Entlüftungsöffnungen in das Schuhinnere gepumpt. Hierbei ist es unerheblich, ob das Versteifungselement entweder direkt formschlüssig oder mit einem geeigneten Kleber stoffschlüssig ohne die Bildung von Hohlräumen mit der Innensohle verbunden wird.

Es ist besonders zweckmäßig, die Luft gleichzeitig über an der Unterseite der Innensohle verlaufende Entlüftungsrillen abzuleiten, um so einen Luftaustausch im Schuhinneren zu ermöglichen.

Für einen effizienten Luftaustausch ist es weiterhin von Vorteil, wenn die Rillen an der Unterseite der Innensohle strahlenförmig von der Wölbung aus auf die Außenkanten der Innensohle zulaufen.

Bei der Rückformung der Wölbung nach ihrer Belastung wird durch die Entlüftungslöcher die Luft aus dem Schuhinneren in die durch den zwischen Wölbung und Schuhinnensohle liegenden Bereich gebildete Luftkammer eingesogen und gleichzeitig durch die Entlüftungsrillen frische Luft von außerhalb in die Luftkammer geleitet. Somit wird ein ständiger Luftaustausch im Schuhinneren ermöglicht. Die Entlüftungslöcher werden zweckmäßig durch Perforation der Schuhinnensohle geschaffen, wobei darauf zu achten ist, dass insbesondere die Entlüftungslöcher, die sich im Bereich der Wölbung befinden nicht durch das darunterliegende Einlegeteil verdeckt werden. Dies kann erreicht werden, indem die Lage der Entlüftungslöcher mit den Aussparungen in des Einlegeteils korrespondiert. Optional werden weitere Entlüftungslöcher im Bereich des Vorfußes eingebracht, welche mit den an der Unterseite der Schuhinnensohle angebrachten Rillen korrespondieren.

Wie beschrieben, lässt sich so der Tragekomfort durch die Ausbildung einer Wölbung an der Innensohle erhöhen und gleichzeitig eine einfache Ventilation des Schuhinnenraums ohne großen materiellen und finanziellen Aufwand erzielen.

Aufgrund der für eine Innensohle vorteilhaften Eigenschaften, wie Flexibilität, Belastbarkeit etc., ist es sinnvoll, die Innensohle aus einem elastischen Kunststoffmaterial zu bilden oder aus einem anderen Material, weclhes die genannten Eigenschaften besitzt.

Insbesondere ist es von Vorteil, wenn es sich bei der Innensohle um eine Einlegesohle handelt, da diese bei Beschädigung oder bei starker Abnutzung gegebenenfalls ausgewechselt werden kann.

Durch eine Textilie an der dem Fuß zugewandten Oberseite der Innensohle kann der Tragekomfort noch weiter erhöht werden.

Besonders wirkungsvoll lässt sich die erfindungsgemäße Innensohle in einem entsprechend angepassten Schuh nutzen. Hierbei ist es sinnvoll, dass der Schuh die Zirkulation der Luft im Schuhinneren, d.h. die Zu- und Ableitung von Luft, über die Entlüftungsrillen in der Innensohle unterstützend ermöglicht.

Besonders vorteilhaft hat sich in diesem Zusammenhang die Verwendung einer gasdurchlässigen Membran zumindest an den am Schuh endenden Stellen der Entlüftungsrillen erwiesen, die einen Luftaustausch des Schuhinneren über die Entlüftungsrillen ermöglicht.

Die Membran sollte das Eindringen von Flüssigkeiten und Schmutz in das Schuhinnere unterbinden sowie die Flüssigkeit aus dem Schuhinneren nach außen durchlassen. Beispielsweise kommen für die Membran GORE-TEX-ähnliche Materialien in Frage. Die Membran ist in vorteilhafter Weise in das Außenmaterial des Schuhs integriert.

Anhand der in den beiliegenden Zeichnungen dargestellten bevorzugten Ausführungsformen wird die Erfindung im Folgenden näher erläutert. Ähnliche oder korrespondierende Einzelheiten sind in den Figuren mit denselben Bezugszeichen versehen. Es zeigen:
Figur 1 die dem Fuß zugewandte Oberseite einer erfindungsgemäßen Innensohle
Figur 2 eine Seitenansicht auf die Innensohle aus Skizze 1,

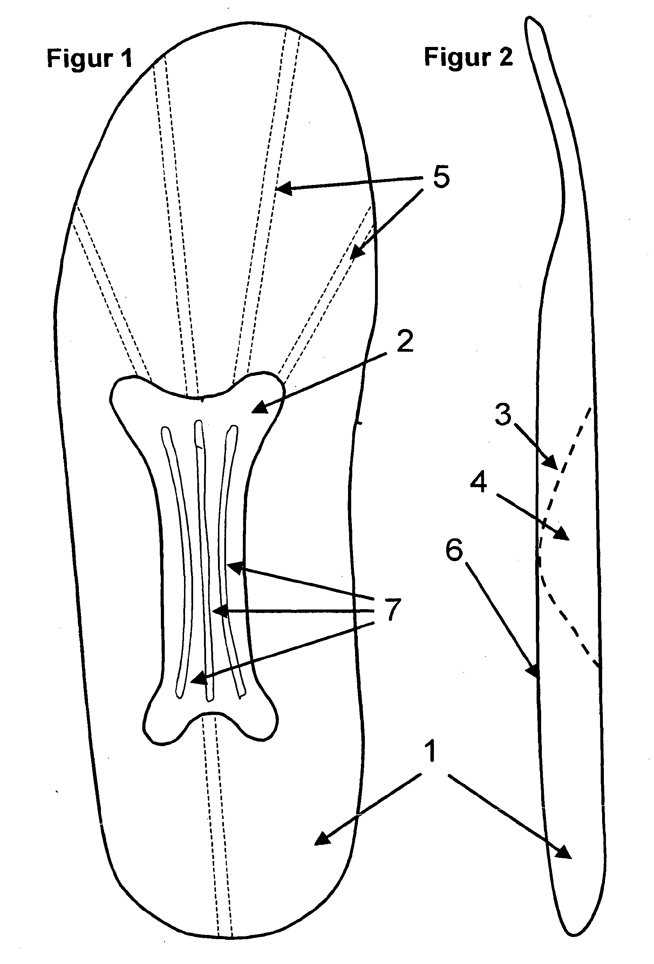

Figur 1 zeigt exemplarisch eine Ansicht auf die dem Fuß zugewandten Oberseite einer erfindungsgemäßen Innensohle 1 sowie des darin eingepassten knochenförmiges Einlegeteil 2. Die Form der Schuhsohle ist dabei im Wesentlichen dem Profil eines menschlichen Fußes angepasst. Wie aus der Seitenansicht aus Figur 2 ersichtlich, besitzt die Innensohle 1 in ihrer Mitte eine Wölbung 3.

An die Wölbung 3 sind mehrere strahlenförmig verlaufende Entlüftungsrillen 5 angeformt. Die Entlüftungsrillen laufen entlang der Unterseite der Innensohle 1 auf den äußeren Rand der Innensohle 1 zu. Des Weiteren ist an der Außenseite der Innensohle 1 eine Randerhöhung 6 vorgesehen, die sich im Wesentlichen zwischen Ballenbereich und Fersenbereich erstreckt. Die Randerhöhung erleichtert das Einsetzen der Innensohle 1 in einen Schuh, falls diese als Einlegesohle ausgebildet ist, und erhöht den Tragekomfort.

Zusätzlich ist an der Oberseite der Innensohle 1 eine dünne durch eine Textilie gebildete Schicht angebracht.

Die dem Fuß zugewandte Wölbung 3 bildet an der Unterseite, dem Schuh zugewandt, eine Luftkammer. Über Rillen 5 kann die Luft aus dem Schuhinnenraum mit dem Schuhäußeren ausgetauscht werden. Dadurch wird eine Belüftung des Schuhinneren erreicht.

Dabei ist es zweckmäßig, dass die Rillen 5 durch eine Belastung durch den menschlichen Fuß beim Gehen nicht so verformbar sind, dass durch sie kein Luftaustausch mehr erfolgen kann. Eine gewisse Steifigkeit der Rillen 5 ist demnach vorzusehen. Des weiteren ist aus Skizze 1 ein Versteifungselement 2 ersichtlich, welches durch seine konvexe Form mit der Wölbung der Innensohle 1 korrespondiert, so dass beide Teile entweder einfach in aufeinander gelegt werden können, so dass eine formschlüssige Verbindung entsteht, oder die beiden Teile werden mit einander verklebt, so dass eine stoffschlüssige Verbindung entsteht.

Wird durch den Fuß beim Gehen ein Druck auf die Innensohle ausgeübt, so verkleinert sich der Hohlraum 4 der Wölbung 3. Während in EP-1 536 710-A1 die Rückkehr in die Ausgangslage der Wölbung ausschließlich durch die Elastizität der Innensohle, unterstützt das dieser Anmeldung zugrunde liegende Versteifungselement 2 die Rückstellung aktiv in dem es aus elastischem Material gefertigt ist, welches federelastische Eigenschaft aufweist.

Das Versteifungselement ist mit längsseitigen Schlitzen versehen, so dass die federelastischen Materialeigenschaften in vorteilhafter Weise unterstützt werden, indem die zwischen den Schlitzen entstehenden Stege zusätzliche Translationsenergie beim Zusammendrücken speichern können, welche bei der Rückstellung wieder frei werden, so dass die Rückstellung schneller erfolgt als in einer Ausführung ohne Versteifungselement.

Da das Versteifungselement auf der fußabgewandten Seite der Innensohle eingesetzt wird, kann es aus stabilem Kunststoff ausgeführt werden, ohne dass der Tragekomfort des Schuhes mit Einlegesohle beeinträchtigt wird.

Das Versteifungselement erhöht somit die Lebensdauer der Einlegesohle, deren eigene federelastische Eigenschaft ohne dieses Versteifungselement einer beschleunigten Abnutzung unterworfen wäre.

## Patentansprüche

1. Innensohle (1), die im Wesentlichen dem Profil eines menschlichen Fußes angepasst ist und im Mittelfußbereich eine dem Fuß zugewandte elastisch verformbare Wölbung (4) aufweist, wobei
sich auf der dem Fuß abgewandten Seite der Innensohle im Bereich der Wölbung ein Versteifungselement (2) befindet, welches
aus einem anderen Material mit höherer Steifigkeit als das Material der Innensohle besteht, und
eine konvexe Form aufweist, **dadurch gekennzeichnet, dass** längsseitige Schlitze (7) in dem Versteifungselement (2) vorgesehen sind, von denen ein mittlerer Schlitz gerade verläuft und die äußeren Schlitze der Form des Versteifungselementes (2) entsprechend leicht nach außen gebogen verlaufen.

2. Innensohle nach Anspruch 1, **dadurch gekennzeichnet, dass** das Versteifungselement mit der Innensohle (1) derart geformt ist, so dass die Konvexität des Versteifungselementes (2) mit der Wölbung der Innensohle korrespondiert.

3. Innensohle nach den Ansprüchen 1-2, **dadurch gekennzeichnet, dass** das Versteifungselement derart angepasst ist, so dass Innensohle (1) und Versteifungselement (2) entweder direkt formschlüssig oder mit einem geeigneten Kleber stoffschlüssig ohne die Bildung von Hohlräumen miteinander verbunden werden können.

4. Innensohle nach einem der Ansprüche 1-3, **dadurch gekennzeichnet, dass** das Versteifungselement durch eine Wölbung in Kuppelform federelastische Eigenschaften aufweist.

5. Innensohle nach einem der Ansprüche 1-4, **dadurch gekennzeichnet, dass** das Versteifungselement aus geformten Kunststoff besteht.

6. Innensohle nach einem der Ansprüche 1-5, **dadurch gekennzeichnet, dass** die Innensohle im Bereich der Wölbung kleine Löcher (3) zur Belüftung aufweist, deren Position derart gewählt wird, dass durch das darunter liegende Versteifungselement der der Zirkulation dienende Luftstrom nicht eingeschränkt wird.

7. Innensohle nach einem der Ansprüche 1-6, **dadurch gekennzeichnet dass** die Innensohle an ihrer Unterseite Rillen (5) aufweist, über die aus dem Schuhinneren ventilierte Luft gegen Außenluft ausgetauscht wird.

8. Innensohle nach einem der Ansprüche 1-7, **dadurch gekennzeichnet, dass** die Entlüftungsrillen (5) im Wesentlichen strahlenförmig von der Wölbung (4) zur Außenkante der Innensohle verlaufen.

9. Innensohle nach einem der Ansprüche 1-8, **dadurch gekennzeichnet, dass** die Innensohle aus einem elastischen Kunststoffmaterial besteht.

10. Innensohle nach einem der Ansprüche 1-9, **dadurch gekennzeichnet, dass** die Innensohle an ihrer dem Fuß zugewandten Seite eine durch eine Textilie gebildete Oberfläche besitzt, welche optional eine Perforation durch kleine Löcher ausweist, derart angeordnet, dass diese die Ventilation unterstützen.

11. Innensohle nach einem der Ansprüche 1-10, **dadurch gekennzeichnet, dass** die Innensohle eine Einlegesohle ist.

12. Schuh mit Innensohle gemäß einem der Ansprüche 1-11.

## Claims

1. An insole (1) which is substantially adapted to the profile of a human foot and comprises, in the mid-foot region, an elastically deformable arch (4) facing the foot, wherein
a reinforcement element (2) is located on the side of the insole facing away from the foot in the region of the arch, said reinforcement element
being made of a different material with a greater stiffness than the material of the insole and
having a convex shape, **characterized in that** longitudinal slits (7) are provided in the reinforcement element (2), of which a central slit extends in straight fashion and the outer slits extend slightly bent to the outside in conformity with the shape of the reinforcement element (2).

2. The insole according to claim 1, **characterized in that** the reinforcement element is formed with the insole (1) such that the convexity of the reinforcement element (2) matches the arch of the insole.

3. The insole according to claims 1-2, **characterized in that** the reinforcement element is adapted such that insole (1) and reinforcement element (2) are interconnectable either directly by form locking or with a suitable adhesive by material locking without the formation of cavities.

4. The insole according to any one of claims 1-3, **characterized in that** the reinforcement element exhibits resilient properties due to a dome-like arch.

5. The insole according to any one of claims 1-4, **characterized in that** the reinforcement element consists of a shaped plastic material.

6. The insole according to any one of claims 1-5, **characterized in that** the insole in the region of the arch comprises small holes (3) for ventilation, the position of said holes being chosen such that the air current that serves circulation is not impeded by the reinforcement element positioned thereunder.

7. The insole according to any one of claims 1-6, **characterized in that** the insole is provided on its underside with grooves (5) through which air ventilated out of the shoe interior is replaced by outside air.

8. The insole according to any one of claims 1-7, **characterized in that** the venting grooves (5) extend substantially radially from the arch (4) to the outer edge of the insole.

9. The insole according to any one of claims 1-8, **characterized in that** the insole is made from an elastic plastic material.

10. The insole according to any one of claims 1-9, **characterized in that** the insole on its side facing the foot has a surface formed by a textile, which is optionally provided with a perforation by way of small holes, arranged such that said holes support ventilation.

11. The insole according to any one of claims 1-10, **characterized in that** the insole is an insertable insole.

12. A shoe with insole according to any one of claims 1-11.

## Revendications

1. Semelle intérieure (1) ou semelle de propreté, qui est sensiblement adaptée au profil d'un pied humain, et présente dans la zone centrale du pied, une voute (4) élastiquement déformable, dirigée vers le pied, semelle intérieure dans laquelle
sur le côté de la semelle intérieure, opposé à celui dirigé vers le pied, dans la zone de la voute, se trouve un élément de rigidification (2), qui
est réalisé en un autre matériau avec une rigidité plus élevée que celle du matériau de la semelle intérieure, et
présente une forme convexe,
**caractérisée en ce que** dans l'élément de rigidification (2) sont prévues des fentes (7) d'orientation longitudinale, dont une fente centrale s'étend de manière rectiligne et les fentes extérieures s'étendent de manière légèrement courbée vers l'extérieur conformément à la forme de l'élément de rigidification (2) .

2. Semelle intérieure selon la revendication 1, **caractérisée en ce que** l'élément de rigidification est façonné ou formé avec la semelle intérieure (2) de manière à ce que la convexité de l'élément de rigidification (2) corresponde à la voute de la semelle intérieure.

3. Semelle intérieure selon les revendications 1-2, **caractérisée en ce que** l'élément de rigidification est adapté de manière telle, que la semelle intérieure (1) et l'élément de rigidification (2) puissent être reliés mutuellement, soit directement par complémentarité de formes, soit, à l'aide d'une colle appropriée, par une liaison de continuité de matière, sans la formation d'espaces creux ou de cavités.

4. Semelle intérieure selon l'une des revendications 1-3, **caractérisée en ce que** l'élément de rigidification présente des propriétés d'élasticité de ressort, grâce à une courbure de voute en forme de coupole.

5. Semelle intérieure selon l'une des revendications 1-4, **caractérisée en ce que** l'élément de rigidification est réalisé en matière plastique façonnée par formage.

6. Semelle intérieure selon l'une des revendications 1-5, **caractérisée en ce que** la semelle intérieure présente, dans la région de la voute, des petits trous (3) pour l'aération, dont la position est choisie de manière à ce que l'élément de rigidification situé en-dessous ne restreigne pas l'écoulement d'air servant à la circulation de l'air.

7. Semelle intérieure selon l'une des revendications 1-6, **caractérisée en ce que** la semelle intérieure présente sur son côté inférieur, des rainures (5) par l'intermédiaire desquelles de l'air ventilé hors de l'intérieur de la chaussure est échangé contre de l'air extérieur.

8. Semelle intérieure selon l'une des revendications 1-7, **caractérisée en ce que** les rainures de ventilation (5) s'étendent sensiblement en forme de rayons, de la voute (4) vers le bord extérieur de la semelle intérieure.

9. Semelle intérieure selon l'une des revendications 1-8, **caractérisée en ce que** la semelle intérieure est réalisée en une matière plastique élastique.

10. Semelle intérieure selon l'une des revendications 1-9, **caractérisée en ce que** la semelle intérieure possède, sur son côté dirigé vers le pied, une surface formée par une matière textile, qui présente, de manière optionnelle, une perforation formée de petits trous agencés de façon à ce que ceux-ci contribuent à la ventilation.

11. Semelle intérieure selon l'une des revendications 1-10, **caractérisée en ce que** la semelle intérieure est une semelle rapportée amovible.

12. Chaussure comprenant une semelle intérieure selon l'une des revendications 1-11.
